# EUROPÄISCHE PATENTSCHRIFT

(11) **EP 1 173 230 B1**
(45) Veröffentlichungstag und Bekanntmachung des Hinweises auf die Patenterteilung: **20.08.2003**
(21) Anmeldenummer: 00929357.2
(22) Anmeldetag: 14.04.2000
(51) Int. Cl.: A61L 2/18, A01N 33/04, A01N 37/44, C11D 3/00

(54) **VERFAHREN ZUR DESINFIZIERENDEN PFLEGE VON FUSSBÖDEN**
METHOD FOR TREATING AND MAINTAINING FLOORS WITH DISINFECTANT
PROCEDE D'ENTRETIEN DESINFECTANT DE SOLS

(30) Priorität: 23.04.1999 DE 19918475
(43) Veröffentlichungstag der Anmeldung: 23.01.2002
(73) Patentinhaber: Ecolab Inc., St. Paul, MN 55102-1390 (US)
(72) Erfinder: BIERING, Holger, D-41516 Grevenbroich (DE); ROGMANN, Karl-Heinz, D-40880 Ratingen (DE); HILTNER, Heiko, D-40880 Ratingen (DE)
(74) Vertreter: Godemeyer, Thomas, Dr.
(86) Internationale Anmeldenummer: EP0003383
(87) Internationale Veröffentlichungsnummer: WO00064496

(56) Entgegenhaltungen:
- DE-A- 4 335 046
- DE-A- 19 603 977
- US-A- 3 976 501
- US-A- 4 652 585

## Beschreibung

Die im folgenden beschriebene Erfindung liegt auf dem Gebiet der Reinigung und Desinfektion von harten Oberflächen, in erster Linie Fußböden, und betrifft insbesondere ein Verfahren, bei dem diese Flächen gleichzeitig desinfiziert und mit einem pflegenden Überzug versehen werden können.

Neben Reinigung und Pflege von Fußböden ist in hygienisch besonders anspruchsvollen Bereichen in mehr oder weniger regelmäßigen Abständen auch eine Desinfektion der Fußböden notwendig, um anhaftende Mikroorganismen möglichst weitgehend zu vernichten. Dies gilt beispielsweise im Bereich der Lebensmittelindustrie und der Großküchen, insbesondere aber im medizinischen Bereich, vor allem in Arztpraxen und Krankenhäusern. Um Arbeitszeit einzusparen, hat es nicht an Versuchen gefehlt, die Reinigung, Pflege und Desinfektion der Fußböden in einem Arbeitsgang durchzuführen. Hierfür sind beispielsweise pflegende Reinigungsmittel entwickelt worden, die Wachse als pflegende Komponente zusammen mit Mikrobiziden aus den Klassen Aldehyde, Phenole oder quartäre Ammoniumverbindungen enthalten. Diese Mittel haben jedoch aus mehreren Gründen bisher nicht befriedigt: Zum einen kommt es bei der Verwendung von Aldehyden und Phenolen wegen des hohen Dampfdrucks dieser Verbindungen zu Geruchsbelästigungen und in besonderen Fällen auch zu inhalationstoxikologischen Risiken. Quartäre Ammoniumverbindungen besitzen zwar diese Nachteile nicht, weisen dafür aber nur ein sehr begrenztes mikrobizides Wirkungsspektrum auf. Zum anderen ergeben Wachse verhältnismäßig weiche und empfindliche Pflegefilme. Aus dem letztgenannten Grund ist man bestrebt, anstelle von Wachsen filmbildende Polymere zu verwenden, die härtere und damit widerstandsfähigere Pflegefilme auf den Fußböden ergeben. Auf dieser Basis sind zahlreiche pflegende Reinigungsmittel und reine Pflegemittel für Fußböden entwickelt worden, die in der Regel anionische Polymere, insbesondere Carboxylgruppen enthaltende Polymere als Pflegekomponente enthalten. Die Kombination dieser Pflegekomponenten mit mikrobiziden Wirkstoffen hat allerdings häufig zu Schwierigkeiten geführt. Insbesondere aminische Desinfektionswirkstoffe erwiesen sich als ausgesprochen unverträglich mit den Polymeren, die als Pflegekomponente in Fußbodenreinigungsmittel allgemein gebräuchlich sind. Gerade einige Vertreter dieser aminischen Desinfektionswirkstoffe besitzen aber besondere Vorteile in Form eines äußerst breiten Wirkungsspektrums, geringer Einsatzkonzentration und geringen Dampfdrucks, so daß aus diesen Gründen ihre Verwendung in desinfizierenden Pflegemitteln wünschenswert gewesen wäre. Die im folgenden beschriebene Erfindung hatte sich zur Aufgabe gesetzt hier neue und bessere Lösungen zu entwickeln.

In der US-A-3,976,501 wird ein Verfahren zum Reinigen und Polieren von Fußböden beschrieben, wobei Formulierungen Verwendung finden, die filmbildende, wasserunlösliche Emulsionscopolymere, Alkali-lösliche Harze sowie antibakterielle Mittel enthalten. Als filmbildende Emulsionscopolymere kommen vorzugsweise in Frage: Copolymere von Acryl- oder Methacrylsäureestern mit C₁₋₄-Alkylmethacrylaten, Acrylnitril, Methacrylnitril, Vinylacetat, Styrol, Vinyltoluol, Vinylchlorid oder Vinylidenchlorid. Bevorzugte Alkali-lösliche Harze sind Copolymere von Styrol mit Maleinsäureanhydrid. Als antibakterielles Mittel wird eine Fülle von bekannten bakteriostatischen Wirkstoffen genannt.

Überraschenderweise wurde gefunden, daß bestimmte aminische Desinfektionswirkstoffe sich zusammen mit bestimmten filmbildenden Polymeren gemeinsam in wäßrigen Zubereitungen zur reinigenden Pflege und Desinfektion von harten Oberflächen, insbesondere von Fußböden, einsetzen lassen.

Gegenstand der Erfindung ist ein Verfahren zur desinfizierenden Pflege von harten Fußböden, bei dem die Fußböden mit einer verdünnten wäßrigen Zubereitung gewischt werden, die mindestens einen mikrobiziden Wirkstoff und mindestens eine pflegende Komponente enthält, und die Fußböden ohne Nachspülen mit Wasser trocknen gelassen werden, wobei es sich bei dem mikrobiziden Wirkstoff um ein Alkylpropylendiamin der allgemeinen Formel I 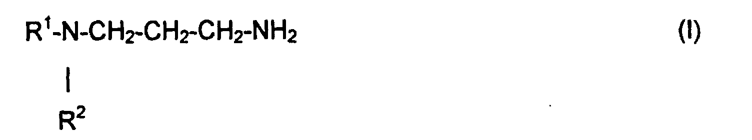 worin R¹ eine Alkyl- oder Alkenylgruppe mit 8 bis 18 Kohlenstoffatomen und R² Wasserstoff, eine Alkylgruppe mit 1 bis 4 Kohlenstoffatomen oder eine Aminoalkylgruppe mit 2 bis 4 Kohlenstoffatomen bedeuten,
und/oder ein Umsetzungsprodukt aus Alkylpropylendiamin der Formel II

R³-NH-CH₂-CH₂CH₂-NH₂ (II)

in der R³ für eine lineare Alkylgruppe mit 12 bis 14 Kohlenstoffatomen steht, mit Verbindungen der Formel III 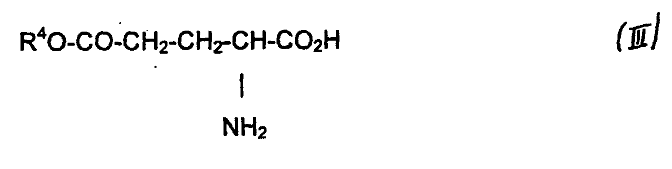 in der R⁴ für Wasserstoff oder eine Alkylgruppe mit 1 bis 4 Kohlenstoffatomen steht, im Molverhältnis 1 : 1 bis 1 : 2 handelt, dadurch gekennzeichnet, daß es sich bei der pflegenden Komponente um ein filmbildendes Acrylat-Copolymer, das 3 bis 60 Mol-% wenigstens eines Aminoalkyl(meth)acrylats als Comonomer enthält, handelt.

Als harte Fußböden werden dabei im Gegensatz zu Teppichböden solche Böden mit Belägen wie Stein, Holz, Linoleum, Kunststoff, Gummi und Keramik angesehen.

Das neue Verfahren erlaubt es, in bisher unerreichter Weise die breit wirksamen antimikrobiellen Wirkstoffe auf Aminbasis ohne Einbuße der Wirksamkeit in einem Verfahren gleichzeitig mit der reinigenden Pflege von Fußböden auszubringen. Mit dem Verfahren werden sehr strapazierfähige Pflegefilme erzeugt und gleichzeitig wird eine anhaltende mikrobizide Wirkung erreicht, die den Anforderungen an eine Flächendesinfektion nach den Prüfkriterien der Deutschen Gesellschaft für Hygiene und Mikrobiologie (DGHM) gerecht wird. Besonders vorteilhaft ist, daß die verwendeten aminischen Mikrobizide mit den genannten Polymeren auch in konzentrierter Form verträglich sind, so daß zur Herstellung der verdünnten wäßrigen Zubereitung wäßrige Konzentrate verwendet werden können, die sämtliche Komponenten im richtigen Verhältnis enthalten und für die eigentliche Anwendung nur noch mit Wasser verdünnt werden müssen. Ein weiterer unerwarteter Vorteil ist darin zu sehen, daß die genannten aminischen Mikrobizide die Reinigungswirkung verstärken.

Bei den im erfindungsgemäßen Verfahren verwendeten Mikrobiziden handelt es sich um Alkylpropylendiamine und/oder Derivate von Alkylpropylendiaminen. Die Mikrobizide sind ausgewählt aus der Gruppe, bestehend aus Alkylpropylendiaminen mit der allgemeinen Formel I 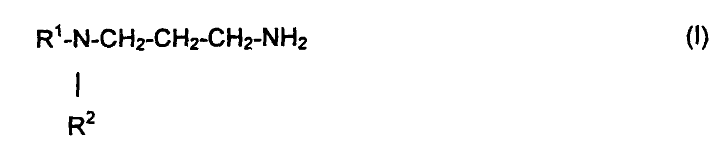 worin R¹ eine Alkyl- oder Alkenylgruppe mit 8 bis 18 Kohlenstoffatomen und R² Wasserstoff, eine Alkylgruppe mit 1 bis 4 Kohlenstoffatomen oder eine Aminoalkylgruppe mit 2 bis 4 Kohlenstoffatomen bedeuten, und den als Glucoprotamin bekannten Produkten, wie sie aus Alkylpropylendiamin der Formel II

R³-NH-CH₂-CH₂CH₂-NH₂ (II)

in der R³ für eine lineare Alkylgruppe mit 12 bis 14 Kohlenstoffatomen steht, durch Umsetzung mit Verbindungen der Formel III 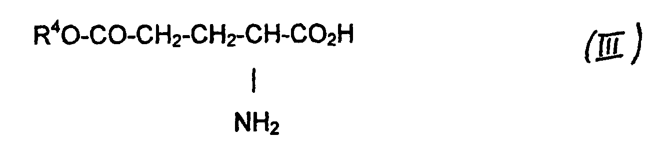 in der R⁴ für Wasserstoff oder eine Alkylgruppe mit 1 bis 4 Kohlenstoffatomen steht, im Molverhältnis 1 : 1 bis 1 : 2 bei 60 bis 175 °C zugänglich sind. Bei den Alkylpropylendiaminen mit der allgemeinen Formel I hat R¹ vorzugsweise die Bedeutung einer linearen Alkylgruppe mit 12 bis 14 Kohlenstoffatomen. R² ist vorzugsweise Wasserstoff oder eine Aminopropylgruppe. Besonders bevorzugte Mikrobizide der allgemeinen Formel I sind N-Laurylpropylendiamin und N,N-Bis-(aminopropyl)-laurylamin. Verbindungen der allgemeinen Formel I sind im Handel erhältlich, beispielsweise unter der Bezeichnung Genamin von der Firma Clariant und unter der Bezeichnung Lonzabac 12 von der Firma Lonza. Besonders bevorzugt werden im Rahmen der vorliegenden Erfindung als Mikrobizide die unter der Bezeichnung Glucoprotamin von der Firma Henkel angebotenen Kondensationsprodukte aus den Verbindungen der Formel II und III, wie sie auch im europäischen Patent 156 275 beschrieben sind.

Selbstverständlich ist es auch möglich, diese aminischen Mikrobizide in Form ihrer Salze mit anorganischen oder organischen Säuren einzusetzen. Dies kann unter Umständen aus Gründen der besseren Löslichkeit vorteilhaft sein.

In dem erfindungsgemäß zur desinfizierenden Pflege der Fußböden verwendeten verdünnten wäßrigen Zubereitungen sollen die Mikrobizide in einer für die Desinfektion ausreichenden Konzentration enthalten sein. Üblicherweise genügen hierzu Konzentrationen von etwa 0,15 Gew.-%. Bevorzugt werden Konzentrationen zwischen etwa 0,05 Gew.-% und etwa 0,3 % an Mikrobizid verwendet, jeweils gerechnet als freies Amin. Die zur Herstellung der verdünnten wäßrigen Zubereitung verwendeten Konzentrate enthalten die aminischen Mikrobizide in entsprechend höherer Konzentration. Vorzugsweise enthalten diese Konzentrate 5 bis 30 Gew.-%, insbesondere 10 bis 20 Gew.-% der aminischen Mikrobizide, ebenfalls gerechnet als freies Amin.

Bei der pflegenden Komponente in den erfindungsgemäß verwendeten verdünnten wäßrigen Zubereitungen handelt es sich um filmbildende Polymere auf Basis von Acrylsäureestem und/oder Methacrylsäureestern, die einen gewissen Anteil an aminogruppenhaltigen Monomeren und gegebenenfalls auch andere nichtcarboxylhaltige Comonomere aufweisen. Im einzelnen handelt es sich um Acrylcopolymere, die etwa 3 bis 60 Mol-%, vorzugsweise etwa 5 bis etwa 40 Mol-% wenigstens eines Aminoalkyl(meth)acrylats als Comonomer enthalten. Vorzugsweise handelt es sich dabei um N-Alkyl- und N,N-Dialkylaminoalkyl(meth)acrylate, in denen die N-Alkylgruppen 1 bis 4 C-Atome aufweisen. Besonders bevorzugt werden als Comonomer Aminoalkyl(meth)acrylate aus der Gruppe Dimethylaminomethylacrylat, Diethylaminomethylacrylat, Dimethylaminoethylacrylat, Diethylaminoethylacrylat, tert-Butylaminoethylacrylat, Dimethylaminoneopentylacrylat, Dimethylaminomethylmethacrylat, Diethylaminomethylmethacrylat, Dimethylaminoethylmethacrylat, Diethylaminoethylmethacrylat, tert-Butylaminoethylmethacrylat und Dimethylaminoneopentylmethacrylat. Obwohl mehrere verschiedene Aminoalkyl(meth)acrylate als Comonomere in den filmbildenden Polymeren enthalten sein können, wird bevorzugt nur ein derartiges Monomer, vorzugsweise aus der obengenannten Gruppe bei der Herstellung der Polymeren verwendet.

Der zweite wesentliche Comonomeranteil der erfindungsgemäß verwendeten filmbildenden Copolymere besteht aus Estem der Acrylsäure und/oder der Methacrylsäure mit Alkoholen, die keine weiteren funktionelle Gruppen aufweisen. Monomere dieses Typs sind in den Polymeren vorzugsweise in Mengen zwischen etwa 40 und etwa 97 Mol-%, insbesondere in Mengen zwischen etwa 60 und etwa 95 Mol-% enthalten. Besonders bevorzugt werden die Ester von Acrylsäure und/oder Methacrylsäure mit solchen aliphatischen Alkoholen, die 1 bis 8 C-Atomen aufweisen. Von diesen Estern wiederum werden Methylacrylat, Ethylacrylat, Propylacrylat, Isopropylacrylat, Butylacrylat, Isobutylacrylat, Hexylacrylat, Heptylacrylat, Octylacrylat, 2-Ethylhexylacrylat, Methylmethacrylat, Ethylmethacrylat, Propylmethacrylat, Isopropylmethacrylat, Butylmethacrylat, Isobutylmethacrylat, Hexylmethacrylat, Heptylmethacrylat, Octylmethacrylat und 2-Ethylhexylmethacrylat besonders bevorzugt. Vorzugsweise enthalten die erfindungsgemäß verwendeten filmbildenden Polymeren wenigstens zwei verschiedene (Meth)acrylsäureester neben den Aminoalkyl(meth)acrylaten.

Prinzipiell können die erfindungsgemäß verwendeten filmbildenden Polymeren weitere Comonomere anderer Struktur enthalten, sofern diese die positiven Eigenschaften der Polymeren nicht wesentlich verändern. Beispiele derartiger Comonomerer sind Styrol, Vinylalkylether, Vinylester, Crotonsäureester, gegebenenfalls substituierte Acryl- oder Methacrylamide und Acrylnitril. Monomere, die anionische Gruppen wie beispielsweise Carboxylgruppen enthalten, sollen in den Polymeren nicht enthalten sein. Vorzugsweise enthalten die erfindungsgemäß verwendeten Polymeren weniger als 20 Mol-%, insbesondere weniger als 10 Mol-% an solchen Comonomeren, die keine (Meth)acrylsäureesterstruktur aufweisen. Die besonders bevorzugten filmbildenden Polymeren sind frei von derartigen Monomeren.

Die erfindungsgemäß verwendeten filmbildenden Polymeren können nach Standardverfahren der Polymerchemie aus den entsprechenden Monomeren durch radikalische Copolymerisation hergestellt werden. Eine Vielzahl derartiger Polymerer ist überdies im Handel für andere Zwecke erhältlich. Vorzugsweise werden diese Polymeren als wäßrige Dispersionen angeboten, die die Polymeren in fein verteilter Form enthalten. Ein Beispiel für ein geeignetes Polymer in dieser Form ist das Produkt WOKAMER K7762 der Firma Worlée.

Die filmbildenden Polymeren werden in den erfindungsgemäß verwendeten verdünnten wäßrigen Zubereitungen üblicherweise in Konzentrationen zwischen etwa 0,2 Gew.-% und etwa 5 Gew.-%, vorzugsweise zwischen etwa 0,3 Gew.-% und etwa 3 Gew.-%, und insbesondere zwischen etwa 1 Gew.-% und etwa 2 Gew.-% eingesetzt. Die Konzentration richtet sich selbstverständlich auch nach der Stärke des Pflegefilms, der mit dem Verfahren erzeugt werden soll. In den zur Herstellung der verdünnten wäßrigen Zubereitung verwendeten Konzentraten liegt der Gehalt an Polymer entsprechend höher, vorzugsweise zwischen 20 Gew.-% und 65 Gew.-%, insbesondere zwischen 35 Gew.-% und 55 Gew.-%. Üblicherweise enthalten die im erfindungsgemäßen Verfahren angewandten wäßrigen Zubereitungen neben den vorgenannten Polymeren keine weiteren filmbildenden Substanzen, doch ist es in Einzelfällen durchaus möglich, zusätzlich derartige Substanzen, wie beispielsweise Wachse, neben den oben genannten Polymeren zu verwenden.

Grundsätzlich müssen die verdünnten wäßrigen Zubereitungen, mit denen die Fußböden im erfindungsgemäßen Verfahren gewischt werden, außer den Mikrobiziden und den filmbildenden Polymeren keine weiteren Komponenten enthalten. Üblicherweise werden diesen Zubereitungen aber weitere Hilfs- und Zusatzstoffe zugefügt, um die Handhabung und die Gebrauchseigenschaften, beispielsweise das Benetzungsverhalten, die Reinigungswirkung, die Gleichmäßigkeit des gebildeten Schutzfilms und die Stabilität der Lösungen selbst zu verbessern. Als Beispiele derartiger Hilfs- und Zusatzstoffe, wie sie auch in anderen Fußbodenpflegemitteln üblich sind, sollen hier nur Tenside, Verlaufshilfsmittel, Komplexbildner, Säuren, organische Lösungsmittel, Lösungsvermittler, Farbstoffe und Duftstoffe genannt werden. Selbstverständlich sollen keine solchen Substanzen zugesetzt werden, die die positiven Eigenschaften des Verfahrens oder die Stabilität der Konzentrate beeinträchtigen.

Tenside dienen im allgemeinen dazu, die Benetzung der Fußböden zu erleichtern und die Reinigungswirkung zu verstärken. Im erfindungsgemäßen Verfahren kommen insbesondere nichtionische und amphotere Tenside oder Mischungen derartiger Tenside zum Einsatz. Als nichtionische Tenside haben sich vor allem Alkylenoxidaddukte als besonders geeignet erwiesen, wie sie durch Anlagerung von 3 bis 30 Mol Ethylenoxid (EO) und/oder Propylenoxid (PO) an Fettalkohole, langkettige Oxoalkohole, Fettsäuren, Fettamine und Alkylphenole mit jeweils 8 bis 18 Kohlenstoffatomen in den Alkylketten erhältlich sind. Die endständigen Hydroxylgruppen dieser Polyglykolether-Derivate können gegebenenfalls auch verethert, verestert oder acetalisiert sein. Besonders geeignet sind Anlagerungsprodukte von 3 bis 15 Mol Ethylenoxid an gesättigte oder ungesättigte Fettalkohole mit 8 bis 18 Kohlenstoffatomen, Anlagerungsprodukte von 3 bis 5 Mol Ethylenoxid und 3 bis 6 Mol Propylenoxid an gesättigte oder ungesättigte Fettalkohole mit 8 bis 18 Kohlenstoffatomen, wobei diese gemischten Alkylenoxidaddukte sowohl im statistischen als auch im Blockpolyadditionsverfahren hergestellt sein können, sowie Etherderivate der vorgenannten Fettalkoholalkylenglykolether, in denen die endständigen Hydroxylgruppen mit einem geradkettigen oder verzweigten gesättigten aliphatischen Alkohol mit vorzugsweise 4 bis 8 Kohlenstoffatomen verethert sind.

Eine weitere Gruppe geeigneter nichtionischer Tenside stellen die Aminoxide von aliphatischen tertiären Monoaminen dar, die wenigstens einen langkettigen Alkyl-, Alkenyl- oder Amidoalkylrest mit 8 bis 18 C-Atomen aufweisen. Beispiele für geeignete Aminoxide sind Bis(2-hydroxyethyl)talgalkylaminoxid (Aromox® T12) und Bis(2-hydroxyethyl)kokosalkylaminoxid (Aromox® C12).

Zu den geeigneten amphoteren Tensiden gehören Derivate tertiärer aliphatischer Amine und quartärer aliphatischer Ammoniumverbindungen, deren aliphatische Reste geradkettig oder verzweigt sein können, und von denen einer eine Carboxy-Sulfo-, Phosphono-, Sulfato- oder Phosphatogruppe trägt. Wenigstens einer der aliphatischen Reste sollte eine langkettige Alkyl-, Alkenyl- oder Amidoalkylgruppe mit 8 bis 18 C-Atomen sein. Beispiele für geeignete amphotere Tenside sind N,N-dimethyl-N-tetradecylglycin, N,N-dimethyl-N-hexadecylglycin, N,N-dimethyl-N-octadecylglycin und 3-(N,N-dimethyl-N-dodecylammonium)-1-propansulfonat.

In Einzelfällen sind auch anionische oder kationische Tenside geeignet. Sie werden aber weniger bevorzugt verwendet. Die Konzentration an Tensiden beträgt in den erfindungsgemäß verwendeten verdünnten wäßrigen Zubereitungen vorzugsweise nicht mehr als etwa 0,25 Gew.-%. Vorzugsweise liegt die Konzentration zwischen etwa 0,03 Gew.-% und etwa 0,2 Gew.-%, insbesondere zwischen etwa 0,05 Gew.-% und etwa 0,18 Gew.-%. In den zur Herstellung der verdünnten wäßrigen Zubereitungen verwendeten Konzentrate liegt der Gehalt an Tensiden vorzugsweise nicht über 15 Gew.-%, insbesondere zwischen 1 Gew.-% und 10 Gew.-%. Wenn mehrere Tenside nebeneinander verwendet werden, bezieht sich diese Mengenangabe auf den Gehalt an Tensiden insgesamt.

Verlaufshilfsmittel, die auch als Koalisziermittel oder Weichmacher bezeichnet werden, dienen zur Verbesserung der Eigenschaften des aus den Polymeren entstehenden Pflegefilms. Es handelt sich bei diesen auch in herkömmlichen Fußbodenpflegemitteln üblichen Substanzen um schwer flüchtige oder nicht flüchtige polare organische Verbindungen. An flüchtigen Verbindungen, die auch als temporäre Weichmacher bezeichnet werden, seien hier beispielsweise Ethylendiglykol, Methyldiglykol und Butyldiglykol genannt. Als permanente Weichmacher eignen sich beispielsweise Dibutylphthalat und Tributoxyethylphosphat. Der Gehalt an Verlaufshilfsmitteln liegt in den verdünnten wäßrigen Zubereitungen üblicherweise nicht über etwa 0,05 Gew.-%, insbesondere zwischen etwa 0,01 Gew.-% und 0,03 Gew.-%. In den zur Herstellung der verdünnten wäßrigen Zubereitung vorzugsweise verwendeten Konzentraten liegt der Gehalt üblicherweise nicht über etwa 5 Gew.-%, insbesondere zwischen etwa 1 Gew.-% und etwa 3 Gew.-%, jeweils bezogen auf die Gesamtmenge an Verlaufshilfsmitteln.

Komplexbildner dienen im erfindungsgemäßen Verfahren in erster Linie der Verbesserung der Reinigungswirkung und der Sequestrierung der Wasserhärte beim Verdünnen. Als Komplexbildner eignen sich vor allem Aminopolycarbonsäuren, Polyphosphonsäuren, Phosphonocarbonsäuren und Hydroxycarbonsäuren. In den erfindungsgemäß verwendeten Zubereitungen und Konzentraten liegen die Komplexbildner in der Regel zumindest teilweise als Salze vor. Vorzugsweise werden Alkalisalze, insbesondere Dinatriumsalze verwendet. Beispiele für geeignete Komplexbildner sind Nitrilotriessigsäure, Ethylendiamintetraessigsäure, 1-Hydroxyethan-1,1-diphosphonsäure, Aminotris-(methylenphosphonsäure), Ethylendiamintetrakis-(methylenphosphonsäure), Phosphonobutantricarbonsäure, Weinsäure, Zitronensäure, Gluconsäure, Methylglycindiessigsäure sowie Derivate der Polyasparaginsäure. Selbstverständlich können auch mehrere Komplexbildner nebeneinander verwendet werden. In der erfindungsgemäß verwendeten verdünnten wäßrigen Zubereitung liegt die Konzentration an Komplexbildnem insgesamt, gerechnet als freie Säuren, üblicherweise nicht über etwa 0,1 Gew.-%, insbesondere zwischen etwa 0,005 Gew.-% und etwa 0,06 Gew.-%. In den zur Herstellung der Zubereitungen verwendeten Konzentraten kann der Gehalt an Komplexbildnern bis zu 10 Gew.-% betragen und liegt insbesondere zwischen 0,5 und 6 Gew.-%.

Zur Herstellung der erfindungsgemäß verwendeten wäßrigen Zubereitungen können auch nicht komplexbildende Säuren verwendet werden, um den gewünschten pH-Wert einzustellen, der vorzugsweise im schwach sauren bis etwa neutralen Bereich liegt. Besonders bevorzugt liegt der pH-Wert des Konzentrats zwischen etwa 4 und etwa 6,5, während der pH-Wert der verdünnten wäßrigen Zubereitung vorzugsweise zwischen etwa 6,5 und etwa 7,5 liegt. Die Menge an Säure, die benötigt wird, richtet sich in erster Linie danach, ob die aminischen Bestandteile in Form der freien Amine oder in Form von Salzen bei der Herstellung der Konzentrate bzw. wäßrigen Zubereitungen eingesetzt werden. Prinzipiell sind alle anorganischen und alle wasserlöslichen organischen Säuren, insbesondere Carbonsäuren, für diesen Zweck geeignet. Besonders bevorzugt wird Essigsäure.

Organische Lösungsmittel können in den erfindungsgemäß verwendeten wäßrigen Zubereitungen zu einer Verstärkung der Reinigungswirkung beitragen, haben aber in der Regel die Aufgabe, in den verwendeten Konzentraten für eine stabile homogene Mischung der Bestandteile zu sorgen.

Geeignet sind wassermischbare, leicht flüchtige organische Lösungsmittel, insbesondere die Monoalkohole mit 1 bis 4 Kohlenstoffatomen, beispielsweise Ethanol und Isopropanol, sowie die leicht flüchtigen Glykolether, soweit diese nicht bereits als Verlaufshilfsmittel gelten können. Der Gehalt an organischen Lösungsmitteln beträgt in den zur Herstellung der verdünnten wäßrigen Zubereitung eingesetzten Konzentrate meist nicht über 15 Gew.-% und liegt insbesondere zwischen 3 und 10 Gew.-%.

Zur Homogenisierung der Konzentrate können anstelle oder zusammen mit den organischen Lösungsmitteln aber auch andere Lösungsvermittler, auch als Hydrotrope bezeichnet, eingesetzt werden, wenn das im Einzelfall notwendig erscheint. Beispiele derartiger Hydrotrope sind kurzkettige Alkylsulfonate und Arylsulfonate, beispielsweise Cumolsulfonat. Die Konzentration richtet sich wie bei allen anderen Hilfs- und Zusatzstoffen nach dem gewünschten Effekt.

Die im erfindungsgemäßen Verfahren eingesetzten verdünnten wäßrigen Zubereitungen können prinzipiell durch getrenntes Auflösen jedes einzelnen darin enthaltenen Wirkstoffs hergestellt werden. Vorzugsweise werden aber mehrere der enthaltenen Wirkstoffe oder insbesondere sämtliche enthaltenen Wirkstoffe zunächst in Form eines Vorkonzentrats vereinigt und die verdünnte wäßrige Zubereitung aus diesem Konzentrat durch Verdünnen mit Wasser und gegebenenfalls Hinzufügen weiterer Wirkstoffe hergestellt. Diese Konzentrate, die die notwendigen Wirkstoffe und Zusatzstoffe im selben Mengenverhältnis enthalten, wie sie in der später verwendeten verdünnten wäßrigen Zubereitung gebraucht werden, lassen sich aus den Wirkstoffen durch Vermischen gegebenenfalls unter Zuhilfenahme von organischen Lösungsmitteln und/oder Lösungsvermittlern gewinnen. Besonders leicht lassen sich diese Konzentrate herstellen, wenn von vorformulierten Polymerdispersionen ausgegangen wird. Die Menge der Wirkstoffe und Zusatzstoffe in den Konzentraten wird in der Regel so gewählt, daß durch Verdünnen dieser Konzentrate mit Wasser im Verhältnis von 1 : 20 bis 1 : 400, vorzugsweise im Verhältnis von 1 : 50 bis 1 : 200 eine gebrauchsfertige verdünnte wäßrige Zubereitung erhalten wird.

Zur Durchführung des erfindungsgemäßen Verfahrens werden die Fußböden mit den verdünnten wäßrigen Zubereitungen gewischt, wobei vorzugsweise etwa 20 ml bis etwa 100 ml pro m² Fußbodenoberfläche möglichst gleichmäßig aufgebracht werden. Das Wischen kann mit Hilfe von weichen, vorzugsweise saugfähigen Gegenständen, beispielsweise Bürsten, Tüchern und Schwämmen vorgenommen werden, und kann manuell oder mit Hilfe geeigneter Maschinen ausgeführt werden. Dabei kann das Aufbringen der verdünnten wäßrigen Zubereitung auch getrennt vom anschließenden Wischvorgang, beispielsweise durch Aufsprühen erfolgen. Wenn eine verstärkte Reinigung gewünscht wird, können auch zunächst größere Mengen an wäßriger Zubereitung ausgebracht und die überschüssigen Mengen nach dem Wischen zusammen mit dem abgelösten Schmutz wieder vom Fußboden aufgenommen werden. Die auf den Fußboden verbleibende Menge an wäßriger Zubereitung läßt man eintrocknen, wobei sich der gewünschte Pflegefilm bildet.

### Beispiele

### Herstellung eines erfindungsgemäßen desinfizierenden Fußbodenpflegemittels (Produkt 4)

15 Gewichtsteile des als Glucoprotamin® bekannten Wirkstoffes (100 % Aktivsubstanz) werden in 33 Gewichtsteilen Wasser unter Erwärmen gelöst. Nach dem Abkühlen auf eine Temperatur < 40 °C wird unter Verwendung von Essigsäure ein pH-Wert von 5,8 - 6,0 in der Lösung eingestellt. Diese Lösung wird mit 50 Gewichtsteilen Wokamer® K 7762 (Fa. Worlee Chemie GmbH, Lübeck) gemischt.

Wokamer® K 7762 ist ein Acrylatcopolymer aus Dimethylminoethylmethacrylat einerseits und C₁- bis C₄-Estern der Acrylsäure andererseits unter Zuhilfenahme von Methylmethacrylat und/oder Styrol zur Erzeugung einer Glastemperatur von 49 °C. Die Aminzahl des Copolymers beträgt 75 mg KOH pro g Polymer; das Produkt stellt eine 30gewichtsprozentige wäßrige Zubereitung on pH 5,2 bis 5,9 dar.

### Allgemeine Vorschrift zur Herstellung der Vergleichs-Produkte 1 und 2

In einem Rührbehälter wird Wasser vorgelegt und mit den Desinfektionswirkstoffen (QAV bzw. Glucoprotamin) bei Raumtemperatur vermischt. Anschließend werden die nichtionischen Tenside, Lösungsmittel und gegebenenfalls Hilfsstoffe zur Mischung gegeben und intensiv gerührt bis eine klare Lösung vorliegt.

### Allgemeine Vorschrift zur Herstellung des Vergleichs-Produktes 3

In einem Rührbehälter wird Wasser vorgelegt und die anionischen und nichtionischen Tenside bei Raumtemperatur gelöst. Anschließend erfolgt unter Rühren die Zugabe des Wokamer® C 3301 (Fa. Worlee Chemie GmbH, Lübeck) sowie die Hilfsstoffe.

### Testmethoden

Die Prüfung der Anwendungseigenschaften eines erfindungsgemäßen Mittels und der Vergleichs-Produkte erfolgte unter Verwendung der nachfolgenden Testmethode:
◆ Ermittlung des Reinigungsverhaltens im Gardner-Test.
◆ Ermittlung der mikrobiologischen Wirksamkeit im Flächentest gemäß den Richtlinien der Deutschen Gesellschaft für Hygiene und Mikobiologie.
◆ Ermittlung des Pflege- und Anschmutzungsverhaltens auf verschiedenen Belägen im Praxistest.

### Zusammensetzung der Produkte und Ergebnisse der anwendungstechnischen Prüfungen

| **Rohstoff/Gehalt** | **Vergleichs- Produkt 1** | **Vergleichs- Produkt 2** | **Vergleichs- Produkt 3** | **Erfindungsge- mäßes Produkt 4** |
|---|---|---|---|---|
| Isopropanol | 2,00% | | | |
| Hilfsstoffe | 6,00% | | | |
| Fa+1,2PO+6,4EO, C10-14 | 5,00% | | | |
| QAV, Dimethylalkyl(C12-14)benzylammoniumchlorid | 15,00% | | | |
| Wasser | 72,00% | 48,00% | 78,00% | 62,50% |
| Glucoprotamin | | 25,00% | | 15,00% |
| Diethylenglykolmonobutylether | | 10,00% | | |
| Ethylenglykolmonophenylether | | 10,00% | | |
| FA, C12-14+9EO-butylether | | 5,00% | | 5,00% |
| FA+10EO, Oleyl/Cetyl, JZ50/55 | | | 5,00% | |
| FAS-Li, Lauryl, C8-12 | | | 10,00% | |
| Alkansulfonat-Na-Salz | | | 1,00% | |
| Polyacrylsäure-Copolymer,wässr.Lösung (1) | | | 3,50% | |
| Kationische Reinacrylatlösung (2) | | | | 15,00% |
| Hilfsstoffe | | 2,00% | 2,50% | 2,50% |
| Reinigungsverhalten 1%ig (3) | - | 0 | + | + |
| Pflege/Anschmutzung (4) | - | - | + | ++ |
| Mikrobizide Wirksamkeit 1%ig (5) | 0 | + | - | + |

| | | | | |
|---|---|---|---|---|
| (1) z. B. Wokamer C 3301 | | | | |
| (2) z. B. Wokamer K 7762 | | | | |
| (3) Standardbezugsgröße: Incidur* | | | | |
| (4) Standardbezugsgröße: Incidur* Durchführung des Praxistestes: Oberflächen werden in der Anwendungskonzentration täglich gewischt und begangen. Einmal wöchentlich wird die Teststrecke abgemustert und mit dem Standard verglichen. | | | | |
| (5) Standardbezugsgröße: Incidin extra* * Handelsprodukte der Firma Henkel-Ecolab Bewertung: 0 = wie Standard + = besser als Standard ++ = deutlich besser als Standard - = schlechter als Standard -- = deutlich schlechter als Standard | | | | |

### Auswertung

Das erfindungsgemäße Produkt zeigte in den anwendungstechnischen Prüfungen bezüglich der Reinigungsleistung, der mikrobiologischen Wirksamkeit und hinsichtlich des Pflege- und Anschmutzungsverhaltens analoge und teilweise bessere Ergebnisse als die zum Vergleich herangezogenen Spezialprodukte. In der Summierung der Eigenschaften zeigte das Beispiel der erfindungsgemäßen Zusammensetzung eine deutliche Überlegenheit zu den Vergleichsrezepturen.

## Patentansprüche

1. Verfahren zur desinfizierenden Pflege von harten Fußböden, bei dem die Fußböden mit einer verdünnten wäßrigen Zubereitung gewischt werden, die mindestens einen mikrobiziden Wirkstoff und mindestens eine pflegende Komponente enthält, und die Fußböden ohne Nachspülen mit Wasser trocknen gelassen werden, wobei es sich bei dem mikrobiziden Wirkstoff um
ein Alkylpropylendiamin der allgemeinen Formel I 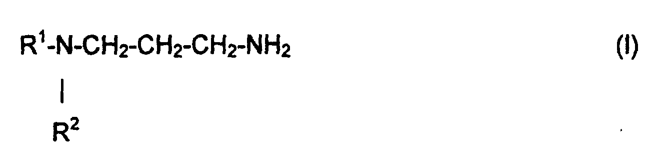 worin R¹ eine Alkyl- oder Alkenylgruppe mit 8 bis 18 Kohlenstoffatomen und R² Wasserstoff, eine Alkylgruppe mit 1 bis 4 Kohlenstoffatomen oder eine Aminoalkylgruppe mit 2 bis 4 Kohlenstoffatomen bedeuten,
und/oder ein Umsetzungsprodukt aus Alkylpropylendiamin der Formel II
R³-NH-CH₂-CH₂CH₂-NH₂ (II)
in der R³ für eine lineare Alkylgruppe mit 12 bis 14 Kohlenstoffatomen steht, mit Verbindungen der Formel III 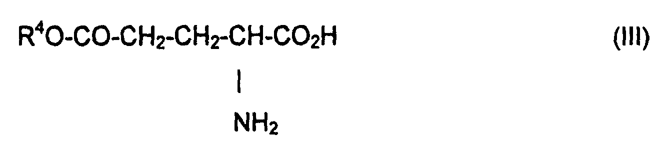 in der R⁴ für Wasserstoff oder eine Alkylgruppe mit 1 bis 4 Kohlenstoffatomen steht, im Molverhältnis 1 : 1 bis 1 : 2 handelt,
**dadurch gekennzeichnet, daß** es sich bei der pflegenden Komponente um ein filmbildendes Acrylat-Copolymer, das 3 bis 60 Mol-% wenigstens eines Aminoalkyl(meth)acrylats als Comonomer enthält, handelt.

2. Verfahren nach Anspruch 1, bei dem als filmbildendes Acrylat-Copolymer ein Copolymer aus a) 5 bis 40 Mol-% an Aminoalkyl(meth)acrylat-Monomeren und b) 95 bis 60 Mol-% an (Meth)acrylsäureestem von Alkoholen mit 1 bis 8 C-Atomen verwendet wird.

3. Verfahren nach einem der Ansprüche 1 oder 2, bei dem das filmbildende Acrylat-Copolymer mindestens ein Monomer aus der Gruppe Dimethylaminomethylacrylat, Diethylaminomethylacrylat, Dimethylaminoethylacrylat, Diethylaminoethylacrylat, tert-Butylaminoethylacrylat, Dimethylaminoneopentylacrylat, Dimethylaminomethylmethacrylat, Diethylaminomethylmethacrylat, Dimethylaminoethylmethacrylat, Diethylaminoethylmethacrylat, tert-Butylaminoethylmethacrylat und Dimethylaminoneopentylmethacrylat und mindestens ein Monomer aus der Gruppe Methylacrylat, Ethylacrylat, Propylacrylat, Isopropylacrylat, Butylacrylat, Isobutylacrylat, Hexylacrylat, Heptylacrylat, Octylacrylat, 2-Ethylhexylacrylat, Methylmethacrylat, Ethylmethacrylat, Propylmethacrylat, Isopropylmethacrylat, Butylmethacrylat, Isobutyimethacrylat, Hexylmethacrylat, Heptylmethacrylat, Octylmethacrylat und 2-Ethylhexylmethacrylat enthält.

4. Verfahren nach einem der Ansprüche 1 bis 3, bei dem als Mikrobizid mindestens ein Wirkstoff aus der Gruppe der als Glucoprotamin bezeichneten Produkte verwendet wird, wie sie aus Alkylpropylendiamin der Formel II
R³-NH-CH₂-CH₂CH₂-NH₂ (II)
in der R³ für eine lineare Alkylgruppe mit 12 bis 14 Kohlenstoffatomen steht, durch Umsetzung mit Verbindungen der Formel III 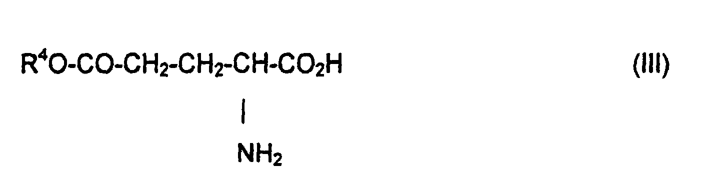 in der R⁴ für Wasserstoff oder eine Alkylgruppe mit 1 bis 4 Kohlenstoffatomen steht, im Molverhältnis 1 : 1 bis 1 : 2 bei 60 bis 175 °C zugänglich sind.

5. Verfahren nach einem der Ansprüche 1 bis 4, bei dem die zur Pflege verwendete wäßrige Zubereitung weitere Hilfs- und Zusatzstoffe aus der Gruppe Tenside, Verlaufshilfsmittel, Komplexbildner, Säuren, organische Lösungsmittel, Lösungsvermittler, Farbstoffe, Duftstoffe und deren Gemische enthält.

6. Verfahren nach einem der Ansprüche 1 bis 5, bei dem die zur Pflege verwendete wäßrige Zubereitung aus einem Konzentrat durch Verdünnen mit Wasser im Verhältnis 1 : 20 bis 1 : 400, vorzugsweise 1 : 50 bis 1 : 200 hergestellt wird.

7. Wäßriges Konzentrat zur Verwendung in einem Verfahren gemäß Anspruch 6, enthaltend
5 bis 30 Gew.-% wenigstens eines Mikrobizids aus der Gruppe der Alkylpropylendiamine mit der allgemeinen Formel I 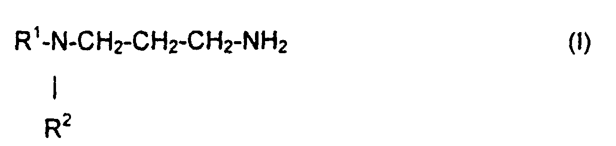 worin R¹ eine Alkyl- oder Alkenylgruppe mit 8 bis 18 Kohlenstoffatomen und R² Wasserstoff, eine Alkylgruppe mit 1 bis 4 Kohlenstoffatomen oder eine Aminoalkylgruppe mit 2 bis 4 Kohlenstoffatomen bedeuten, und der als Glucoprotamin bekannten Produkte, wie sie aus Alkylpropylendiamin der Formel II
R³-NH-CH₂-CH₂CH₂-NH₂ (II)
in der R³ für eine lineare Alkylgruppe mit 12 bis 14 Kohlenstoffatomen steht, durch Umsetzung mit Verbindungen der Formel III 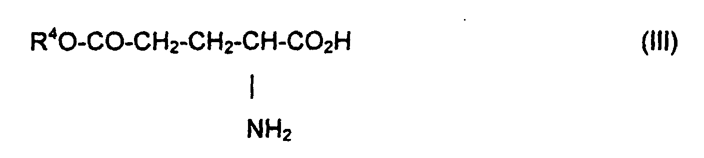 in der R⁴ für Wasserstoff oder eine Alkylgruppe mit 1 bis 4 Kohlenstoffatomen steht, im Molverhältnis 1 : 1 bis 1 : 2 bei 60 bis 175 °C zugänglich sind, **dadurch gekennzeichnet, daß** es
20 bis 65 Gew.-% wenigstens eines filmbildenden Acrylat-Copolymers, das 5 bis 60 Mol-% wenigstens eines Aminoalkyl(meth)acrylats als Comonomer enthält, sowie ferner
0 bis 15 Gew.-% Tensid
0 bis 15 Gew.-% organische Lösungsmittel
0 bis 10 Gew.-% Komplexbildner
enthält.

8. Wäßriges Konzentrat gemäß Anspruch 7, enthaltend
10 bis 20 Gew.-% wenigstens eines Mikrobizids aus der Gruppe der Alkylpropylendiamine mit der allgemeinen Formel I 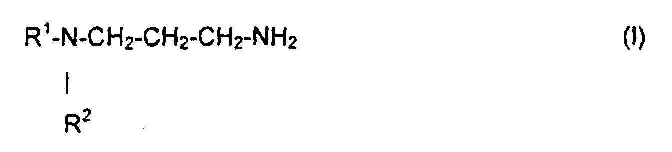 worin R¹ eine Alkyl- oder Alkenylgruppe mit 8 bis 18 Kohlenstoffatomen und R² Wasserstoff, eine Alkylgruppe mit 1 bis 4 Kohlenstoffatomen oder eine Aminoalkylgruppe mit 2 bis 4 Kohlenstoffatomen bedeuten, und der als Glucoprotamin bekannten Produkte, wie sie aus Alkylpropylendiamin der Formel II
R³-NH-CH₂-CH₂CH₂-NH₂ (II)
in der R³ für eine lineare Alkylgruppe mit 12 bis 14 Kohlenstoffatomen steht, durch Umsetzung mit Verbindungen der Formel III 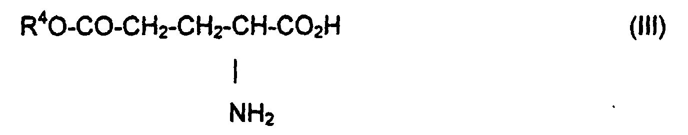 in der R⁴ für Wasserstoff oder eine Alkylgruppe mit 1 bis 4 Kohlenstoffatomen steht, im Molverhältnis 1 : 1 bis 1 : 2 bei 60 bis 175 °C zugänglich sind, **dadurch gekennzeichnet, daß** es
35 bis 55 Gew.-% wenigstens eines filmbildenden Acrylat-Copolymers, das 5 bis 60 Mol-% wenigstens eines Aminoalkyl(meth)acrylats als Comonomer enthält, sowie femer
1 bis 10 Gew.-% Tensid
3 bis 10 Gew.-% organische Lösungsmittel
0,5 bis 6 Gew.-% Komplexbildner
enthält.

9. Wäßriges Konzentrat nach einem der Ansprüche 7 oder 8, enthaltend als Mikrobizid wenigstens einen Wirkstoff aus der Gruppe der als Glucoprotoamin bekannten Produkte, wie sie aus Alkylpropylendiamin der Formel II
R³-NH-CH₂-CH₂CH₂-NH₂ (II)
in der R³ für eine lineare Alkylgruppe mit 12 bis 14 Kohlenstoffatomen steht, durch Umsetzung mit Verbindungen der Formel III 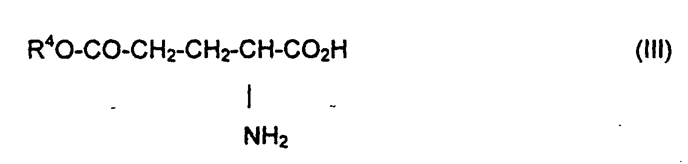 in der R⁴ für Wasserstoff oder eine Alkylgruppe mit 1 bis 4 Kohlenstoffatomen steht, im Molverhältnis 1 : 1 bis 1 : 2 bei 60 bis 175 °C zugänglich sind.

10. Wäßriges Konzentrat nach einem der Ansprüche 7 bis 9, enthaltend wenigstens ein Tensid aus der Gruppe Alkoxylate, Aminoxide und amphotere Tenside.

## Claims

1. A process for the disinfecting care of hard floors in which the floors are wiped with a dilute water-based preparation which contains at least one microbicidal agent and at least one care component and are then left to dry without rinsing with water, the microbicidal agent being an alkyl propylenediamine corresponding to general formula I: 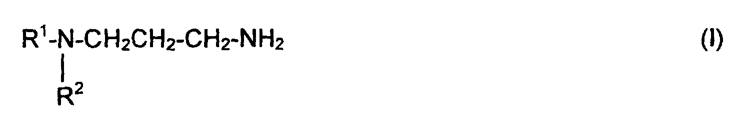 in which R¹ is an alkyl or alkenyl group containing 8 to 18 carbon atoms and R² is hydrogen, an alkyl group containing 1 to 4 carbon atoms or an aminoalkyl group containing 2 to 4 carbon atoms,
and/or a reaction product of an alkyl propylenediamine corresponding to formula II:
R³-NH-CH₂-CH₂CH₂-NH₂ (II)
in which R³ is a linear alkyl group containing 12 to 14 carbon atoms, with compounds corresponding to formula III: 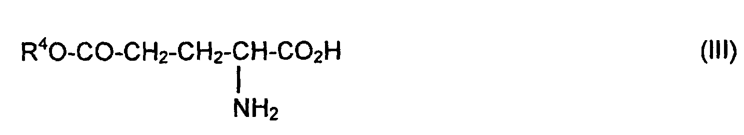 in which R⁴ is hydrogen or an alkyl group containing 1 to 4 carbon atoms, in a molar ratio of 1:1 to 1:2,
**characterized in that** the care component is a film-forming acrylate copolymer containing 3 to 60 mol-% of at least one aminoalkyl (meth)acrylate as comonomer.

2. A process as claimed in claim 1, in which a copolymer of a) 5 to 40 mol-% of aminoalkyl (meth)acrylate monomers and b) 95 to 60 mol-% of (meth)acrylates of alcohols containing 1 to 8 carbon atoms is used as the film-forming acrylate copolymer.

3. A process as claimed in claim 1 or 2, in which the film-forming acrylate copolymer contains at least one monomer from the group consisting of dimethyl aminomethyl acrylate, diethyl aminomethyl acrylate, dimethyl aminoethyl acrylate, diethyl aminoethyl acrylate, tert.butyl aminoethyl acrylate, dimethyl aminoneopentyl acrylate, dimethyl aminomethyl methacrylate, diethyl aminomethyl methacrylate, dimethyl aminoethyl methacrylate, diethylaminoethyl methacrylate, tert.butyl aminoethyl methacrylate and dimethylaminoneopentyl methacrylate and at least one monomer from the group consisting of methyl acrylate, ethyl acrylate, propyl acrylate, isopropyl acrylate, butyl acrylate, isobutyl acrylate, hexyl acrylate, heptyl acrylate, octyl acrylate, 2-ethylhexyl acrylate, methyl methacrylate, ethyl methacrylate, propyl methacrylate, isopropyl methacrylate, butyl methacrylate, isobutyl methacrylate, hexyl methacrylate, heptyl methacrylate, octyl methacrylate and 2-ethylhexyl methacrylate.

4. A process as claimed in any of claims 1 to 3, in which the microbicide used is at least one active substance from the group of products known as glucoprotamine which are obtainable from alkyl propylenediamine corresponding to formula II:
R³-NH-CH₂-CH₂CH₂-NH₂ (II)
in which R³ is a linear alkyl group containing 12 to 14 carbon atoms, by reaction with compounds corresponding to formula III: 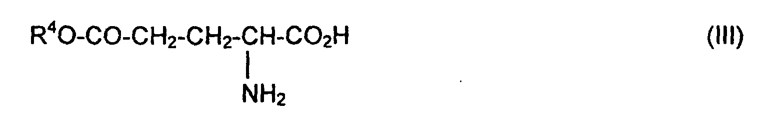 in which R⁴ is hydrogen or an alkyl group containing 1 to 4 carbon atoms, in a molar ratio of 1:1 to 1:2 at temperatures of 60 to 175°C.

5. A process as claimed in any of claims 1 to 4, in which the water-based care preparation contains other auxiliaries and additives from the group consisting of surfactants, flow controllers, complexing agents, acids, organic solvents, solubilizers, dyes, perfumes and mixtures thereof.

6. A process as claimed in any of claims 1 to 5, in which the water-based care preparation is prepared from a concentrate by dilution with water in a ratio of 1:20 to 1:400 and preferably 1:50 to 1:200.

7. A water-based concentrate for use in the process claimed in claim 6 containing
5 to 30% by weight of at least one microbicide from the group of alkyl propylenediamines corresponding to general formula I: 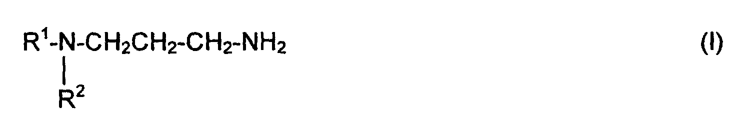 in which R¹ is an alkyl or alkenyl group containing 8 to 18 carbon atoms and R² is hydrogen, an alkyl group containing 1 to 4 carbon atoms or an aminoalkyl group containing 2 to 4 carbon atoms,
and the products known as glucoprotamine which are obtainable from alkyl propylenediamine corresponding to formula II:
R³-NH-CH₂-CH₂CH₂-NH₂ (II)
in which R³ is a linear alkyl group containing 12 to 14 carbon atoms,
by reaction with compounds corresponding to formula III: 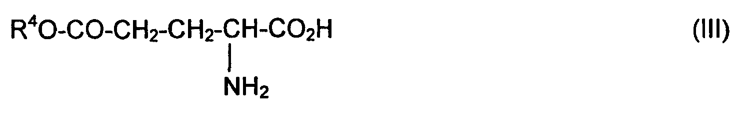 in which R⁴ is hydrogen or an alkyl group containing 1 to 4 carbon atoms, in a molar ratio of 1:1 to 1:2 at temperatures of 60 to 175°C, **characterized in that** it contains
20 to 65% by weight of at least one film-forming acrylate copolymer containing 5 to 60 mol-% of at least one aminoalkyl (meth)acrylate as comonomer and
0 to 15% by weight of surfactant,
0 to 15% by weight of organic solvents and
0 to 10% by weight of complexing agents.

8. A water-based concentrate as claimed in claim 7 containing
10 to 20% by weight of at least one microbicide from the group of alkyl propylenediamines corresponding to general formula I: 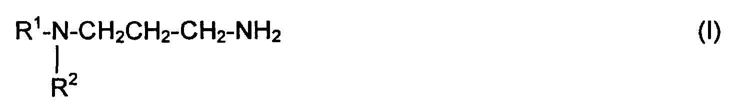 in which R¹ is an alkyl or alkenyl group containing 8 to 18 carbon atoms and R² is hydrogen, an alkyl group containing 1 to 4 carbon atoms or an aminoalkyl group containing 2 to 4 carbon atoms,
and the products known as glucoprotamine which are obtainable from alkyl propylenediamine corresponding to formula II:
R³-NH-CH₂-CH₂CH₂-NH₂ (II)
in which R³ is a linear alkyl group containing 12 to 14 carbon atoms,
by reaction with compounds corresponding to formula III: 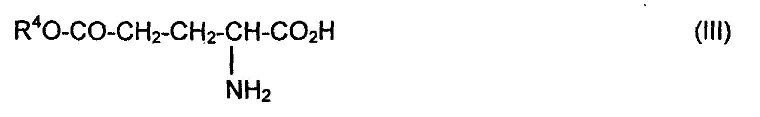 in which R⁴ is hydrogen or an alkyl group containing 1 to 4 carbon atoms,
in a molar ratio of 1:1 to 1:2 at temperatures of 60 to 175°C, **characterized in that** it contains
35 to 55% by weight of at least one film-forming acrylate copolymer containing 5 to 60 mol-% of at least one aminoalkyl (meth)acrylate as comonomer and
1 to 10% by weight of surfactant,
3 to 10% by weight of organic solvents and
0.5 to 6% by weight of complexing agents.

9. A water-based concentrate as claimed in claim 7 or 8 containing as microbicide at least one active substance from the group of products known as glucoprotamine which are obtainable from alkyl propylenediamine corresponding to formula II:
R³-NH-CH₂-CH₂CH₂-NH₂ (II)
in which R³ is a linear alkyl group containing 12 to 14 carbon atoms, by reaction with compounds corresponding to formula III: 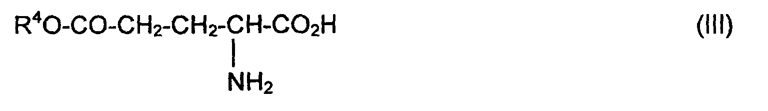 in which R⁴ is hydrogen or an alkyl group containing 1 to 4 carbon atoms, in a molar ratio of 1:1 to 1:2 at temperatures of 60 to 175°C.

10. A water-based concentrate as claimed in any of claims 7 to 9 containing at least one surfactant from the group of alkoxylates, amine oxides and amphoteric surfactants.

## Revendications

1. Procédé d'entretien désinfectant de sols durs, selon lequel on frotte les sols avec une préparation aqueuse diluée qui renferme au moins un principe actif microbicide et au moins un composant d'entretien, et on laisse sécher les sols sans rinçage ultérieur avec de l'eau, le principe actif microbicide étant une alkylpropylènediamine de formule générale I, 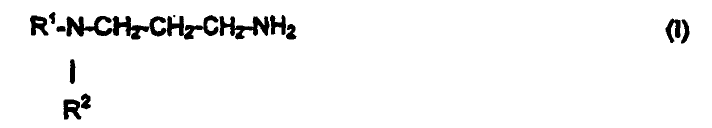 dans laquelle R¹ signifie un groupe alkyle ou alkényle ayant de 8 à 18 atomes de carbone, et R² signifie de l'hydrogène, un groupe alkyle ayant de 1 à 4 atomes de carbone ou un groupe aminoalkyle ayant de 2 à 4 atomes de carbone,
et/ou un produit de réaction à partir d'alkylpropylènediamine de formule II
R³-NH-CH₂-CH₂CH₂-NH₂ (II)
dans laquelle R³ représente un groupe alkyle linéaire ayant de 12 à 14 atomes de carbone, avec des composée de formule III 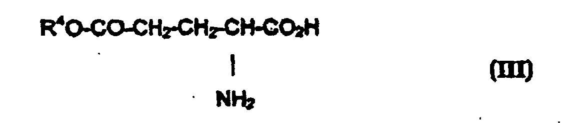 dans laquelle R⁴ représente de l'hydrogène ou un groupe alkyle ayant de 1 à 4 atomes de carbone, dans le rapport molaire 1 : 1 à 1 : 2,
**caractérisé en ce que**
le composant d'entretien est un copolymère d'acrylate filmogène qui renferme comme comonomère de 3 à 60 % molaire d'au moins un (mèth)acrylate d'aminoalkyle.

2. Procédé selon la revendication 1,
dans lequel
comme copolymère d'acrylate filmogène, on utilise un copolymère à base de :
a) 5 à 40 % molaire de monomères de (méth)acrylate d'aminoalkyle, et
b) 95 à 60 % molaire d'esters d'acide (méth)acrylique avec des alcools de 1 à 8 atomes de carbone.

3. Procédé selon l'une quelconque des revendications 1 ou 2,
dans lequel le copolymère d'acrylate filmogène, renferme au moins un monomère choisi dans le groupe de l'acrylate de diméthylaminométhyle, l'acrylate de diéthylaminométhyle, l'acrylate de diméthylaminoéthyle, l'acrylate de diéthylaminoéthyle, l'acrylate de tert-butylaminoéthyle, l'acrylate de diméthylaminonéopentyle, le méthacrylate de diméthylaminométhyle, le méthacrylate de diéthylaminométhyle, le méthacrylate de diméthylaminoéthyle, le méthacrylate de diéthylaminoéthyle, le méthacrylate de tert-butylaminoéthyle, le méthacrylate de diméthylaminonéopentyle et au moins un monomère choisi dans le groupe de l'acrylate de méthyle, l'acrylate d'éthyle, l'acrylate de propyle, l'acrylate d'isopropyle, l'acrylate de butyle, l'acrylate d'isobutyle, l'acrylate d'hexyle, l'acrylate d'heptyle, l'acrylate d'octyle, l'acrylate de 2-éthylhexyle, le méthacrylate de méthyle, le méthacrylate d'éthyle, le méthacrylate de propyle, le méthacrylate d'isopropyle, le méthacrylate de butyle, le méthacrylate d'isobutyle, le méthacrylate d'hexyle, le méthacrylate d'heptile, le méthacrylate d'octyle et le méthacrylate de 2-éthylhexyle.

4. Procédé selon l'une quelconque des revendications 1 à 3,
dans lequel on utilise comme microbicide, au moins un principe actif choisi dans le groupe des produits désignés comme glucoprotamine, ainsi qu'ils sont accessibles à partir d'alkylpropylènediamine de formule II,
R³-NH-CH₂-CH₂CH₂-NH₂ (II)
dans laquelle R³ représente un groupe alkyle linéaire ayant de 12 à 14 atomes de carbone, par réaction avec des composés de formule III 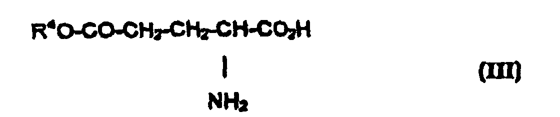 dans laquelle R⁴ représente de l'hydrogène ou un groupe alkyle ayant de 1 à 4 atomes de carbone,
dans un rapport molaire allant de 1 : 1 à 1 : 2 à une température allant de 60 à 175°C.

5. Procédé selon l'une quelconque des revendications 1 à 4,
dans lequel la préparation aqueuse utilisée pour les soins, contient d'autres adjuvants et additifs choisis dans le groupe des agents tensioactifs, des adjuvants d'écoulement, des agents formateurs de complexe, des acides, des solvants organiques, des faciliteurs de mise en solution, des colorants, des parfurns et leurs mélanges.

6. Procédé selon l'une quelconque des revendications 1 à 5,
dans lequel la préparation aqueuse utilisée pour l'entretien est produite à partir d'un concentré par dilution avec de l'eau dans un rapport de 1 : 20 à 1 : 400, de préférence de 1 : 50 à 1 : 200.

7. Concentré aqueux pour l'utilisation dans un procédé conformément à la revendication 6, contenant :
de 5 à 30 % en poids d'au moins un microbicide choisi dans le groupe des alkylpropylènediamines ayant la formule générale I 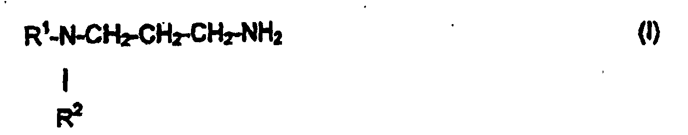 dans laquelle R¹ signifie un groupe alkyle ou alkényle ayant 8 à 18 atomes de carbone, et R² signifie de l'hydrogène, ou groupe alkyle ayant de 1 à 4 atomes de carbone ou un groupe aminoalkyle ayant de 2 à 4 atomes de carbone, et des produits connus comme glucoprotamine, ainsi qu'ils sont accessibles à partir des alkylpropylènediamines de formule II
R³-NH-CH₂-CH₂CH₂-NH₂ (II)
dans laquelle R³ représente un groupe alkyle linéaire ayant de 12 à 14 atomes de carbone, par réaction avec des composés de formule (III) 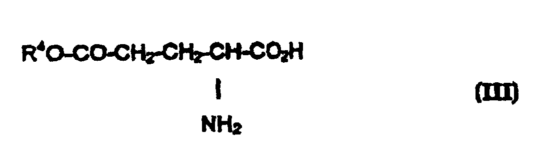 dans laquelle R⁴ représente un hydrogène ou un groupe alkyle ayant de 1 à 4 atomes de carbone,
dans un rapport molaire allant de 1 : 1 à 1 : 2 à une température de 60 à 175°C.
**caractérisé en ce qu'**
il contient de 20 à 65 % en poids d'au moins un copolymère d'acrylate filmogène qui renferme comme co-monomère de 5 à 60 % molaire d'au moins un (méth)acrylate d'aminoalkyle, ainsi qu'en outre :
de 0 à 15 % en poids d'agent tensioactif,
de O à 15 % en poids de solvant organique,
de 0 à 10 % en poids d'agent complexant.

8. Concentré aqueux conformément à la revendication 7,
contenant :
de 10 à 20 % en poids d'au moins un microbicide choisi dans le groupe des alkylpropylènediamines ayant la formule générale I 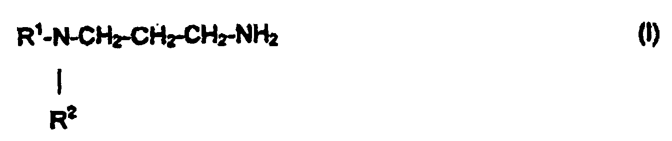 dans laquelle R¹ signifie un groupe alkyle ou alkényle ayant de 8 à 18 atomes de carbone, et R² signifie de l'hydrogène, un groupe alkyle ayant de 1 à 4 atomes de carbone ou un groupe aminoalkyle ayant de 2 à 4 atomes de carbone, et des produits connus comme glucoprotamine, ainsi qu'ils sont accessibles à partir d'alkylpropylènediamine de formule II,
R³-NH-CH₂-CH₂CH₂-NH₂ (II)
dans laquelle R³ représente un groupe alkyle linéaire ayant de 12 à 14 atomes de carbone, par réaction avec des composés de formule III 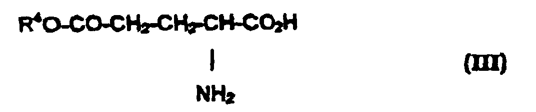 dans laquelle R⁴ représente de l'hydrogène, ou un groupe alkyle ayant de 1 à 4 atomes de carbone, dans un rapport molaire de 1 : 1 à 1 : 2. à une température de 60 à 175°C,
**caractérisé en ce qu'**
il contient de
35 à 55 % en poids d'au moins un copolymère d'acrylate filmogène qui renferme comme monomère au moins de 5 à 60 % molaire d'au moins un (méth)acrylate d'aminoalkyle, ainsi qu'en outre :
de 1 à 10 % en poids d'agent tensioactif
de 3 à 10 % en poids d'un solvant organique
de 0,5 à 6 % en poids d'un agent complexant.

9. Concentré aqueux selon l'une quelconque des revendications 7 ou 8, contenant comme microbiclde au moins un principe actif choisi dans le groupe des produits connus comme glucoprotoamines, ainsi qu'ils sont accessibles à partir d'une alkylpropylènediamine de formule II
R³-NH-CH₂-CH₂CH₂-NH₂ (II)
dans laquelle R³ représente un groupe alkyle linéaire ayant de 12 à 14 atomes de carbone, par réaction avec des composés de formule III 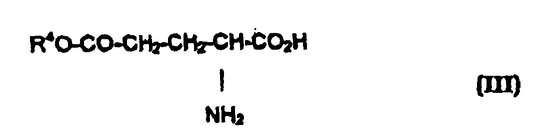 dans laquelle R⁴ représente de l'hydrogène, ou un groupe alkyle ayant de 1 à 4 atomes de carbone, dans un rapport molaire allant de 1 : 1 à 1 : 2 à une température de 60 à 175°C.

10. Concentré aqueux selon l'une quelconque des revendications 7 à 9, contenant au moins un agent tensioactif choisi dans le groupe des alkoxylates, des aminooxydes et des agents tensioactifs amphotères.
